(19) Europäisches Patentamt
European Patent Office
Office européen des brevets

(11) Veröffentlichungsnummer: **0 000 916**
**B1**

# (12) EUROPÄISCHE PATENTSCHRIFT

(45) Veröffentlichungstag der Patentschrift: **20.05.81**

(21) Anmeldenummer: **78100666.3**

(22) Anmeldetag: **16.08.78**

(51) Int. Cl.³: **C 07 C 120/08,**
C 07 C 31/22,
C 07 C 121/14

## (54) Verfahren zur Herstellung von Fettsäurenitrilen und Glycerin aus Glyceriden, insbesondere aus natürlichen Fetten und Oelen.

(30) Priorität: **20.08.77 DE 2737607**
**25.03.78 DE 2813204**

(43) Veröffentlichungstag der Anmeldung:
**07.03.79 Patentblatt 79/5**

(45) Bekanntmachung des Hinweises auf die Patenterteilung:
**20.05.81 Patentblatt 81/20**

(84) Benannte Vertragsstaaten:
**BE CH DE FR GB LU NL SE**

(56) Entgegenhaltungen:
**DE - A - 1 914 688**
**FR - A - 864 571**
**GB - A - 451 594**
**GB - A - 477 463**
**GB - A - 654 462**
**US - A - 2 493 637**

(73) Patentinhaber: **HOECHST AKTIENGESELLSCHAFT**
**Postfach 80 03 20**
**D-6230 Frankfurt/Main 80 (DE)**

(72) Erfinder: **Billenstein, Siegfried, Dr.**
**Samerbergweg 8**
**D-8261 Burgkirchen/Alz (DE)**
Erfinder: **Kukla, Bruno**
**Kalkweg 11**
**D-8261 Gendorf (DE)**
Erfinder: **Stühler, Herbert, Dr.**
**Hochstaufenstrasse 5**
**D-8261 Burgkirchen/Alz (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 000 916 B1

Courier Press, Leamington Spa, England.

Verfahren zur Herstellung von Fettsäurenitrilen und Glycerin aus Glyceriden, insbesondere aus natürlichen Fetten und Ölen

Die Erfindung betrifft ein Verfahren zur Umsetzung von Mono-, Di- und Triglyceriden der allgemeinen Formel

$$\begin{array}{l} CH_2\text{—}O\text{—}(COR_1,H) \\ \mid \\ CH\text{—}O\text{—}(COR_2,H) \\ \mid \\ CH_2\text{—}O\text{—}COR_3 \end{array}$$

worin $R_1$, $R_2$ und $R_3$, die gleich oder verschieden sein können, aliphatische Kohlenwasserstoffreste mit 3 bis 23 Kohlenstoffatomen bedeuten, wobei diese Reste gegebenenfalls mit einer OH-Gruppe substituiert sein können, oder von Gemischen solcher Glyceride in flüssiger Phase mit Ammoniak unter Gewinnung von Fettsäurenitrilen der Formel $R_3$ ($R_1$, $R_2$)—CN und von Glycerin mittels Hindurchleiten von gasförmigem Ammoniak, gegebenenfalls unter Zumischung von Inertgas, bei erhöhten Temperaturen und in Gegenwart von Katalysatoren.

Fettsäurenitrile, die wichtige Zwischenprodukte zur Herstellung von Aminen darstellen, werden nach technischen Verfahren bevorzugt aus den entsprechenden Fettsäuren und Ammoniak in Gegenwart geeigneter Katalysatoren hergestellt. Diese seit langem bekannte Synthese kann sowohl in der flüssigen Phase in einem Temperaturbereich von 250 bis 350°C als auch in der Gasphase in einem Temperaturbereich von 320 bis 380°C durchgeführt werden. Geeignete Katalysatoren für die Reaktion in der flüssigen Phase sind z.B. Zinkoxid oder Manganacetat, für die Gasphase beispielsweise Aluminiumoxid oder Bleicherde. Zusammenfassende Angaben über solche Verfahren finden sich in "Methoden der Organischen Chemie" (Houben-Weyl), Band 8, Seite 330 ff., Georg Thieme Verlag, Stuttgart, 1952, sowie in "Fatty Acids and their Industrial Applications", Marcel Dekker, New York, 1968, Seite 909 ff. Ein Verfahren zur Herstellung von Fettsäurenitrilen durch Umsetzung von Fettsäuren mit Ammoniak in flüssiger Phase in Gegenwart von Zink- oder Calciumsalzen von Fettsäuren beschreibt die US—A 2 589 232. Ein weiteres Verfahren, bei dem Titansäureester kurzkettiger aliphatischer Alkohole als Katalysatoren eingesetzt und neben Fettsäuren auch die Fettsäureester kurzkettiger Alkohole als Ausgangsprodukte verwendet werden können, ist beschrieben in der US—A 2 993 926. Sollen für die genannten Verfahren natürliche Fette oder Öle eingesetzt werden, so bedingt dies zunächst die Gewinnung der freien Fettsäure oder der kurzkettigen Fettsäureester in einer gesonderten Stufe.

Es ist ferner bekannt, natürliche Fette oder auch Fettsäureester einwertiger Alkohole durch Umsetzung mit Ammoniak zu den entsprechenden Fettsäureamiden umzusetzen. Geeignete Katalysatoren hierfür sind beispielsweise Metalloxide oder Calciumnitrat (vgl. japanische Patentbekanntmachungen 70—35524 und 71—21846 sowie 71—6614). Aus den Fettsäureamiden kann dann in bekannter Weise durch Dehydratisierung in einer zweiten Stufe das Fettsäurenitril gewonnen werden.

Aus der japanischen Patentbekanntmachung 72—26921 ist ein Verfahren bekannt, in dem natürliche Fette durch Umsetzung mit aliphatischen Aminen in Fettsäureamide überführt werden können, wobei das entstehende Glycerin durch die Gegenwart von Borsäure als Glycerinborsäureester aus dem Gleichgewicht entfernt wird.

Unter wirtschaftlichen Gesichtspunkten wäre ein Verfahren erstrebenswert, bei dem natürliche Fette oder Öle, das heißt native Triglyceride, die geringe Mengen von Mono- und Diglyceriden enthalten können, ohne Isolierung der Fettsäureamidstufe bzw. ohne den Umweg über die freie Fettsäure oder den kurzkettigen Fettsäureester direkt in die entsprechenden Fettsäurenitrile überführt werden können.

Derartige Versuche sind bereits gemacht worden. In den GB—A 416 613 und 451 594 wird ein Verfahren zur Umsetzung von Hydroxyfettsäuren bzw. Fettsäuren, erhalten aus natürlichen Fetten, mit Ammoniak zu Fettsäurenitrilen in der Gasphase bei Temperaturen von 300 bis 450°C in Gegenwart von oxidischen Dehydratisierungs-Katalysatoren, insbesondere in Gegenwart von Aluminiumoxid, beschrieben. Dort finden sich auch Hinweise, daß Glyceride direkt einsetzbar sind, obwohl diese unter den angegebenen Bedingungen nicht verdampfbar sind. Im Beispiel 3 der GB—A 416 631 und in den Beispielen 7 und 14 der GB—A 451 594 werden solche Umsetzungen von Castoröl, Palmkernfett und Cocosöl mit Ammoniak bei Temperaturen von 350 bis 400°C in Gegenwart von Bauxit gezeigt. Es fehlt hier jedoch jeglicher Hinweise auf den Verbleib des Glycerins. Nacharbeitungen haben ergeben, daß das Glycerin unter den Bedingungen dieses Verfahrens durch thermische Spaltung völlig zersetzt wird.

Dies führt nicht nur zum Verlust eines wertvollen Rohstoffs, sondern bewirkt auch eine entscheidende Verschlechterung der Qualität der als Produkte erhaltenen Fettsäurenitrile, die sich insbesondere in Ferbe und Geruch manifestiert. Da Fettsäurenitrile Zwischenprodukte darstellen und für die Weiterverarbeitung hohe Reinheitsgrade gefordert werden, macht dies aufwendige Reinigungsoperationen zur Beseitigung der Zersetzungsprodukte erforderlich, wodurch das genannte Verfahren völlig unwirtschaftlich wird.

Aus der GB—A 477 463 ist ein Verfahren bekannt, nach dem die Umsetzung von Fettsäuren zu

Nitrilen in flüssiger Phase bevorzugt ohne Katalysatoren durchgeführt werden soll. Dabei wird behauptet, daß beim Einsatz von Glyceriden als Ausgangsmaterial auch Glycerin gewonnen werden kann. Eine experimentelle Nachprüfung ergibt jedoch, daß lediglich geringste Spuren an Glycerin neben einer hohen Menge an Zersetzungsprodukten des Glycerins entstehen, wobei letztere das gebildete Nitril verunreinigen. Aus der US-Patentschrift 2 493 637 ist der Einsatz von Kobaltsalzen von Fettsäuren zur Umsetzung von freien Fettsäuren in Nitrile bekannt. Die französische Patentschrift 864 571 nennt für die Umsetzung von Adipinsäureamid mit Essigsäureanhydrid zu Adiponitril als Katalysatoren unter anderem die Carbonsäuresalze von Schwermetallen.

Somit bestand die Aufgabe, ein einstufiges Verfahren zu entwickeln, nach dem es möglich ist, aus Glyceriden in wirtschaftlich vertretbaren Reaktionszeiten neben dem Fettsäurenitril auch das Glycerin in hoher Ausbeute zu gewinnen.

Die Aufgabe wird im Sinne der Erfindung gelöst durch ein Verfahren zur Umsetzung von Mono-, Di- und Triglyceriden der allgemeinen Formel

$$\begin{array}{l} CH_2—O—(COR_1,H) \\ | \\ CH—O—(COR_2,H) \\ | \\ CH_2—O—COR_3 \end{array}$$

worin $R_1$, $R_2$ und $R_3$, die gleich oder verschieden sein können, gesättigte oder ein- oder mehrfach ungesättigte Kohlenwasserstoffreste mit 3 bis 23 Kohlenstoffatomen bedeuten, wobei diese Reste gegebenenfalls mit einer OH-Gruppe substituiert sein können, oder Gemischen solcher Glyceride in flüssiger Phase mit Ammoniak unter Gewinnung von Fettsäurenitrilen der Formel $R_3$ ($R_1$, $R_3$)—CN und von Glycerin mittels Hindurchleiten von gasförmigem Ammoniak, gegebenenfalls unter Zumischung von Inertgas, bei erhöhten Temperaturen und in Gegenwart von Katalysatoren, das durch gekennzeichnet ist, daß das Glycerid mit einem Ammoniakstrom von mindestens 200 l/kg Glycerid und Stunde, bei Temperaturen von 220 bis 300°C, in Gegenwart von Blei-, Zink-, Cadmium-, Zinn-, Titan-, Zirkon-, Chrom-, Antimon-, Mangan-, Eisen-, Nickel- oder Kobaltsalzen von Carbonsäuren oder Sulfonsäuren als Katalysatoren in innigen Kontakt gebracht, das erhaltene Produktgemisch unter rascher Entfernung des gebildeten Glycerins aus der Reaktionszone ausgetragen und das ausgetragene Produktgemisch einer Phasentrennung in eine Fettsäurenitrilphase und eine Glycerin/Wasser-Phase unterworfen wird.

Ausgangsstoffe für das erfindungsgemäße Verfahren sind Mono-, Di- und Triglyceride der allgemeinen Formel

$$\begin{array}{l} CH_2—O—(COR_1,H) \\ | \\ CH—O—(COR_2,H) \\ | \\ CH_2—O—COR_3 \end{array}$$

das heißt, in dieser Formel können ein oder zwei Fettsäurereste durch H ersetzt sein. Die Fettsäurereste $R_1CO$—, $R_2CO$— und $R_3CO$— leiten sich ab von Fettsäuren mit 3 bis 23 Kohlenstoffatomen. Die aliphatischen Kohlenwasserstoffreste $R_1$, $R_2$ und $R_3$ können geradkettig oder auch ein- oder mehrfach verzweigt sein. Sie können gesättigte Ketten darstellen oder auch eine oder mehrere Mehrfachbindungen, vorzugsweise Doppelbindungen, aufweisen. Gegebenenfalls können diese Reste mit einer OH-Gruppe substituiert sein. Bevorzugte Ausgangsstoffe für das erfindungsgemäße Verfahren sind vor allem die natürlichen Fette, die Gemische aus überwiegend Triglyceriden und kleinen Anteilen aus Diglyceriden und Monoglyceriden darstellen, wobei auch diese meist wiederum Gemische darstellen und verschiedenartige Fettsäurereste im obengenannten Bereich, insbesondere solche mit 8 und mehr C-Atomen, enthalten. Beispielsweise seien genannt pflanzliche Fette, wie Olivenöl, Cocosfett, Palmkernfett, Babussuöl, Palmöl, Erdnußöl, Rüböl, Rizinusöl, Sesamöl, Cottonöl, Sonnenblumenöl, Sojaöl, Hanföl, Mohnöl, Avocadoöl, Baumwollsaatöl, Weizenkeimöl, Maiskeimöl, Kürbiskernöl, Traubenkernöl, Kakaobutter oder Pflanzentalge, ferner tierische Fette, wie Rindertalg, Schweinefett, Knockenfett, Hammeltalg, Japantalg, Walöl und andere Fischöle sowie Lebertran. Ebenso eingesetzt werden können aber auch einheitliche Tri-, Di- und Monoglyceride, sei es, daß diese aus natürlichen Fetten isoliert oder auf synthetischem Wege gewonnen wurden. Hier seien beispielsweise genannt: Tributyrin, Tricapronin, Tricaprylin, Tricaprinin, Trilaurin, Trimyristin, Tripalmitin, Tristearin, Triolein, Trielaidin, Trilinoliin, Trilinolenin, Monopalmitin, Monostearin, Monoolein, Monocaprinin, Monolaurin, Monomyristin oder gemischte Glyceride wie beispeilsweise Palmitodistearin, Distearoolein, Dipalmitoolein oder Myristopalmitostearin.

Entscheidend für das Gelingen des einstufigen Verfahrens, das heißt die Gewinnung von Fettsäurenitril oder einem Gemisch von Fettsäurenitrilen in hoher Reinheit und hoher Ausbeute sowie die Gewinnung von Glycerin in hoher Ausbeute, sind vor allem drei Verfahrensparameter, nämlich die Wahl der richtigen Temperatur bzw. die richtige Temperaturführung, die rasche Entfernung des gebildeten

Glycerins aus der Reaktionszone sowie die Auswahl des richtigen Katalysators.

Die rasche Entfernung des Glycerins aus der Reaktionszone bedingt, daß eine bestimmte Mindestmenge an Ammoniak durch das Reaktionsgemisch hindurchgeleitet wird. Diese beträgt mindestens 200 l pro kg Glycerid und Stunde, vorzugsweise mindestens 400 l pro kg Glycerid und Stunde. Nach oben besteht keine kritische Grenze bezüglich des durchzuleitenden Ammoniakstroms, die mengenmäßige Obergrenze wird allenfalls durch wirtschaftliche Überlegungen bestimmt und liegt aus diesen Gründen bei etwa 1000 l, vorzugsweise bei etwa 800 l Ammoniak pro kg Glycerid und Stunde. Der hindurchgeleiteten Gasmenge kann dabei mit Vorteil bis zu 30%, vorzugsweise bis zu 15%, bezogen auf die durchgesetzte Menge an Ammoniak, an Inertgas, beispielsweise Stickstoff, hinzugefügt werden.

Weiterhin ist es von entscheidender Bedeutung, daß die Reaktionstemperatur während des gesamten Reaktionsverlaufs im Bereich zwischen 220 und 300°C, vorzugsweise 230 bis 290°C, gehalten wird. Vorzugsweise läßt man die Temperatur vom Beginn bis zum Ende des Prozesses ansteigen. Dies kann sowohl kontinuierlich als auch stufenweise, insbesondere in Form eines Temperaturprogramms geschehen. Ein bevorzugtes Temperaturprogramm ist, daß die Reaktion zunächst im Temperaturbereich von etwa 220 bis 240°C lange abläuft, bis etwa 30 bis 70% der theoretisch zu erwartenden Glycerinmenge aus dem Reaktionsgefäß ausgetragen sind, daß man die Temperatur dann im Verlauf von etwa einer halben bis 5 Stunden stufenweise oder kontinuierlich erhöht, bis ein Temperaturbereich von etwa 270 bis 400°C erreicht ist, und die Reaktion dann in diesem Temperaturbereich zu Ende führt. Das Ende der Reaktion ist daran erkennbar, daß keine flüssige Phase mehr in die Vorlage übergeht.

Schließlich ist die Auswahl der Katalysatoren von wesentlicher Bedeutung für das erfindungsgemäße Verfahren. Als Katalystoren können eingesetzt werden die Blei-, Zink-, Cadmium-, Zinn-, Titan-, Zirkon-, Chrom-, Antimon-, Mangan-, Eisen-, Nickel- oder Kobaltsalze von organischen Carbon- oder Sulfonsäuren. Die genannten Katalysatoren können auch in Form von Gemischen Anwendung finden. Die organischen Carbon- und Sulfonsäuren, von denen sich die Anionen der genannten Salze ableiten, gehören insbesondere folgenden Gruppen an:

Offenkettige Carbonsäuren mit gerader oder verzweigter Kette, wobei diese Kette gesättigt ist oder auch eine oder mehrere Doppelbindungen enthalten kann und 4 bis 23, vorzugsweise 8 bis 23 Kohlenstoffatome besitzt,

Alkylcarbonsäuren vorzugsweise Alkylbenzolcarbonsäuren und Alkylnaphthalincarbonsäuren, die einen oder mehrere Alkylreste mit je 1 bis 23, vorzugsweise 1 bis 12 Kohlenstoffatome tragen,

Alkylsulfonsäuren und Alkyldisulfonsäuren mit geradkettigen oder verzweigten Alkylresten mit 4 bis 24, vorzugsweise 8 bis 24 Kohlenstoffatomen, wobei diese alkylreste gegebenenfalls mit OH-Gruppen, vorzugsweise mit einer OH-Gruppe substituiert sein können und wobei ferner in der Alkylkette die Wasserstoffatome teilweise oder vorzugsweise völlig durch Fluor ersetzt sein können, sowie Alkylarylsulfonsäuren, insbesondere Alkylbenzolsulfonsäuren und Alkylnaphthalinsulfonsäuren (wobei hierunter jeweils die Mono-, Di- und Trisulfonsäuren verstanden werden), mit einem oder mehreren geradkettigen oder verzweigten Alkylresten mit je 1 bis 24 Kohlenstoffatomen, wobei die genannten Alkylketten auch mit zwei, vorzugsweise mit einer Carboxylgruppe substituiert sein können.

Von diesen, als Katalysatoren im Rahmen des erfindungsgemäßen Verfahrens einsetzbaren Salzen sind besonders bevorzugt hinsichtlich des Kations die Blei-, Cadmium-, Eisen-, Kobalt- und insbesondere die Zinksalze. Hinsichtlich des Anions sind besonders bevorzugt die obengenannten Gruppen von Sulfonsäuren, insbesondere jedoch daraus wieder die Alkylarylsulfonsäuren der letztgenannten Gruppe. Beispielsweise seien genannt:

n-Dodecylsulfonsäure, n-Octadecylsulfonsäure, Isobutylnaphthalinsulfonsäure, n-Butylnaphthalinsulfonsäure, Isopropylnaphthalinsulfonsäure, Cetylbenzolsulfonsäure, Octadecylbenzolsulfonsäure, Tetrapropylenbenzolsulfonsäure und Triisobutylbenzolsulfonsäure.

Die als Katalysatoren im erfindungsgemäßen Verfahren eingesetzten Salze sind durch Umsetzung der freien Säuren mit den entsprechenden Metalloxiden nach bekannten Verfahren zugänglich. Die freien Sulfonsäuren können erhalten werden aus den bekannten Sulfonierungsprozessen oder aus den entsprechenden Alkalisulfonaten, beispielsweise über Ionenaustauscher.

Die genannten Katalysatoren können in Form ihrer genannten Salze direkt zugesetzt werden. Es kann aber auch das entsprechende Metalloxid und die entsprechende Carbon- oder Sulfonsäure einzeln dem Reaktionsgemisch zugesetzt werden, wobei sich der Katalysator während der Reaktion in situ bildet. Die genannten Katalysatoren werden im erfindungsgemäßen Verfahren in Mengen von 0,5 bis 75 Gew.-%, bei diskontinuierlicher Fahrweise vorzugsweise von 1 bis 25 Gew.-% und insbesondere von 1 bis 10 Gew.-%, bei kontinuierlicher Fahrweise in Mengen von vorzugsweise 5 bis 75 Gew.-%, insbesondere von 10 bis 30 Gew.-%, jeweils bezogen auf das eingesetzte Glycerid, zugegeben.

Zur Durchführung des erfindungsgemäßen Verfahrens wird das jeweilige Glycerid oder Glyceridgemisch in einem geeigneten Reaktionsgefäß, beispielsweise einem Rührkessel, zusammen mit dem oben beschriebenen Katalysator vorgelegt. Es kann die Gesamtmenge an Katalysator vorgelegt werden oder auch eine Teilmenge, wobei der restliche Teil dann während der Reaktion portionsweise oder kontinuierlich nachgeschleust wird. Das Reaktionsgefäß ist mit einer Gaseinleitungsvorrichtung, die die Messung des Gasstromes gestattet, versehen, ferner mit einer Temperaturmeßeinrichtung, einer

Heizeinrichtung und gegebenenfalls mit einem Rührer. Das Reaktionsgefäß ist verbunden mit einer Kondensationsvorrichtung, die aus einer oder vorzugsweise aus mehreren, auf Temperaturen von etwa 60° bis 120°C beheizten Kondensationsvorlagen besteht. Nach dem Aufheizen des Reaktionsgefäßes, das gegebenenfalls unter Stickstoffspülung erfolgt, wird nach Erreichung der Anfangstemperatur der Ammoniakstrom eingestellt. Das in die Kondensationsvorrichtung austretende Produktgemisch besteht in der Anfangsphase aus höheren Anteilen von Rohglycerin. Der Anteil an Fettsäurenitril nimmt im Laufe der Reaktion mehr und mehr zu, so daß im letzten Teil im wesentlichen reines Fettsäurenitril ausgetragen wird, während die Austragung des Rohglycerins bereits vorher beendet ist. Der Ammoniakstrom (dem Inertgas zugefügt sein kann, wie oben beschrieben) sorgt ferner für eine rasche Austragung des Reaktionswassers während der gesamten Reaktionsdauer. Der austretende Gasstrom wird zweckmäßigerweise, nach Abtrennung des mitgeführten Wassers und gegebenenfalls unter Zuführung von frischem Ammoniak, in die Reaktion zurückgeführt. In der Kondensationsvorrichtung sammelt sich das ausgetragene Produktgemisch, wobei eine Vortrennung dieses Gemisches in eine Fettsäurenitrilphase und in eine Rohglycerin/Wasser-Phase erfolgt. Die Abschließende Phasentrennung wird zweckmäßigerweise nach Ablassen der Kondensationsvorrichtung und Überführung in einen Separator, beispielsweise in einen Dampfabscheider, bei etwa 60 bis 100°C vorgenommen. Restliches Glycerin wird mittels Wasser aus der Fettsäurenitrilphase ausgewaschen. Das aus der wäßrigen Phase (beispielsweise durch Destillation) isolierte Rohglycerin kann nach bekannten Verfahren (vergleiche Ullmann Encyklopädie der Technischen Chemie, 1956, Band 7, Seiten 523 bis 524), beispielsweise durch Destillation, gereinigt werden.

Nach dem erfindungsgemäßen Verfahren gelingt es, Fettsäurenitrile und Glycerin in ausgezeichneten Ausbeuten herzustellen. Diese Ausbeuten liegen bei mindestens 93% und erreichen 96% und mehr Fettsäurenitril sowie bis zu 95% Rohglycerin (jeweils bezogen auf die theoretische Ausbeute auf Basis des eingesetzten Glycerids). Das erhaltene Fettsäurenitril enthält höchstens bis zu 2 Gew.-% an freien Fettsäuren und bis zu 10 Gew.-%, meistens aber weniger als 6 Gew.-% Fettsäureamide als Nebenprodukte.

Diese im ausgetragenen Produktgemisch enthaltenen Fettsäuren und Fettsäureamide werden zweckmäßigerweise ebenfalls noch in Fettsäurenitrile überführt, indem sie nach oder gegebenenfalls vor der Phasentrennung einer Nachreaktion in Gegenwart von Ammoniak und in Gegenwart der oben definierten Katalysatoren unterworfen werden.

Dieser Nachreaktion können entweder die aus dem ausgetragenen Fettsäurenitril abgetrennten freien Fettsäuren und Fettsäureamide, gegebenenfalls zusammen mit noch nicht ausgetragenem Rohnitril, unterworfen werden, oder es wird die gesamte Fettsäurenitrilphase nachbehandelt. Dies kann insbesondere nach folgenden Ausführungsformen der Nachreaktion geschehen:

Nach einer bevorzugten Methode wird zwischen den Reaktor und die Kondensationsvorrichtung eine Fraktionierkolonne zwischengeschaltet. Diese Fraktionierkolonne, die eine Spiegelglaskolonne oder ein gut isoliertes Rohr, gefüllt beispielsweise mit Raschigringen, sein kann, trennt die während der Reaktion intermediär gebildeten Fettsäureamide und freien Fettsäuren ab und führt sie in das Reaktionsgefäß zurück, während das restliche ausgetragene Produktgemisch in die Vorlage gelangt und dort unter Kondensation gesammelt wird wie oben beschrieben.

Unter Zwischenschaltung dieses Fraktioniersystems kann das erfindungsgemäße Verfahren in besonders einfacher Weise vollkontinuierlich gestaltet werden. Dazu wird das Glycerid, wie oben beschrieben, zusammen mit den früher genannten Anteilen an Katalysator im Reaktionsgefäß vorgelegt oder wahlweise zu einem inerten, nicht flüchtigem Lösungsmittel, wie beispielsweise Paraffinöl, zudosiert. Die zugeführte Ammoniakmenge soll auch hier mindestens 200 l/kg Glycerid und Stunde betragen und liegt normalerweise bei höheren Durchsätzen als sie oben für die diskontinuierliche Verfahrensweise angegeben wurden.

Die Verfahrenstemperaturen sollen vorteilhafterweise im Temperaturbereich von 220 bis 270°C, insbesondere im Temperaturbereich von 230 bis 250°C gehalten werden. Während der reaktion wird dann das Glycerid kontinuierlich nachgespeist.

Glycerid und Katalysator können auch im Gemisch kontinuierlich zugeführt werden. Über das Fraktioniersystem erfolgt der stetige Austrag von Fettsäurenitril, Glycerin und Wasser, währen Fettsäureamid und freie Fettsäure kontinuierlich in den Prozeß zurückgeführt werden.

Nach einer weiteren Variante der Nachbehandlung wird die Reaktion zunächst bis zur im wesentlichen vollständigen Austragung des Rohglycerins geführt (was bei der Phasentrennung daran erkennbar ist, da die Rohglycerin-Phase nicht mehr zunimmt), sodann wird die aus der Phasentrennung erhaltene Fettsäurenitrilphase zu dem noch nicht ausgetragenen Anteil an Fettsäurenitril zurück in den Reaktor gegeben, wobei im Reaktor die Temperatur auf einen Bereich von 200° bis 320°C ingestellt und der Ammoniakstrom auf eine Menge von 5 bis 150 l/kg Fettsäurenitril und Stunde, vorzugsweise auf 15 bis 100 l/kg, reduziert wird. Sofern im ersten Teil der Reaktion bereits genügend Katalysator eingesetzt worden ist (Katalysatormengen, die an der oberen Grenze der oben angeführten Mengenbereiche liegen), erübrigt sich die neuerliche Zugabe an Katalysator. Andernfalls ist eine entsprechende Menge an Katalysator nachzuführen. Bei diesen Reaktionsbedingungen geht die vollständige Umwandlung von Fettsäuremiden und Fettsäuren in Fettsäurenitrile vor sich, die an der Bildung von Reaktionswasser verfolgt werden kann. Dieses Wasser wird mit dem überschüssigen Ammoniak

ausgetragen, wobei der Ammoniakstrom, gegebenenfalls unter Zugabe von frischen Ammoniak und nach Befreiung von Wasser, wieder in den Prozeß zurückgeführt werden kann. Nach Beendigung der Umsetzung wird das im Reaktor befindliche Fettsäurenitril zur Abtrennung des Katalysators zweckmäßigerweise noch einer Destillation unterworfen.

Nach einer weiteren Methode kann aber auch das gesamte Fettsäurenitril aus der Reaktion ausgetragen, der Phasentrennung unterworfen und dann in einen zweiten Reaktor (Nachreaktor) eingebracht werden, wobei die oben angegebenen Katalysatoren in den genannten Mengen zuzusetzen sind. Die Nachbehandlung erfolgt dann unter den gleichen Bedingungen, wie sie vorstehend bei der Rückführung einer Teilmenge in das erste Reaktionsgefäß genannt wurden. Auch hier ist nach Beendigung der Nachgehandlung das Fettsäurenitril destillativ aufzuarbeiten.

Diese Methode der Nachbehandlung kann auch so abgewandelt werden, daß die gesamte Fettsäurenitrilphase kontinuierlich, beispeilsweise durch einen Rohrreaktor, unter den angegebenen Bedingungen geführt wird.

Schließlich kann die gewonnene Fettsäurenitrilphase auch im gasförmigen Zustand gemeinsam mit Ammoniak in einer Menge von 200 bis 800 l/kg Fettsäurenitril und Stunde, vorzugsweise 300 bis 600 l/kg, und bei einer Temperatur von 280 bis 400°C, vorzugsweise bei 300 bis 380°C, kontinuierlich über ein Festbett von Dehydratisierungskatalysatoren geleitet werden. Geeignete Dehydratisierungskatalysatoren sind beispielsweise Aluminiumoxid in Form von Bauxit oder Hydrargilit, Thoriumoxid, Zirkonoxid, Aluminiumphosphat, Silicagel, aktive Bleicherden und dergleichen oder Mischungen davon.

Nach dem erfindungsgemäßen Verfahren ist es also überraschenderweise möglich, nicht nur Fettsäurennitrile in ausgezeichneten Ausbeuten und guter Reinheit zu gewinnen, sondern es kann auch Glycerin in hohen Ausbeuten gewonnen werden. Mit den geschilderten Nachbehandlungsmethoden kann die Reinheit des gewonnenen Fettsäurenitrils weiter erhöht werden, so daß es weniger als 2 Gew.-%, in den meisten Fällen weniger als 0,1 Gew.-% Fettsäureamid und weniger als 1,5 Gew.-%, in den meisten Fällen weniger als 0,1 Gew.-% freie Fettsäuren enthält und auch sonst von Nebenprodukten völlig frei ist.

Fettsäurenitrile sind wichtige chemische Zwischenprodukte, die insbesondere zu primären Aminen und quaternären Ammoniumsalzen weiterverarbeitet werden, welche wiederum insbesondere als Textilhilfsmittel, Flotationshilfsmittel und als kationenaktive oberflächenaktive Substanzen in vielen technischen Prozessen verwendet werden können. Glycerin ist eine wichtige chemische Verbindung, die beispielsweise für die Herstellung von Sprengstoffen, als Zusatz zu Wärme- und Kraftübertragungsflüssigkeiten, als feuchtigkeitserhaltender Zusatz zu Hautcremes, Zahnpasten, Seifen, Tabak und ähnlichem, als Textilfhilfsmittel, als Lösungsmittel und auf vielen anderen Gebieten, die dem Fachmann bekannt sind, verwendet werden kann.

Das erfindungsgemäße Verfahren wird durch folgende Beispiele erläutert:

## Beispiel 1

Die Reaktion wurde durchgeführt in einem beheizbaren Reaktor von 800 $cm^3$ Inhalt, versehen mit Gaseinleitungsvorrichtung, Rührer, Innenthermometer sowie einer Fraktionierkolonne in Form eines mit Raschigringen gefüllten Glasrohres (Länge 20 cm, Durchmesser 1,5 cm) und eines aus drei hintereinander geschalteten Vorlegen bestehenden Vorlagensystems, in dem die flüchtigen Reaktionsprodukte kondensiert werden. In dieser Apparatur wurden 495 g Talg (Verseifungszahl 190, Säurezahl 7,6) zusammen mit 5 g Zink-Dodecylbenzolsulfonat als Katalysator eingebracht. Während des Aufheizens wurde die Apparatur mit Stickstoff gespült. Anschließend wurde der Stickstoff durch Ammoniakgas ersetzt, wobei 600 l $NH_3$/kg Talg · h im Kreis geführt wurden. Dabei wurde während der Reaktion laufend frisches Ammoniakgas nachgeführt. Im Reaktor ergab sich folgender Temperaturverlauf:

| Reaktionszeit (h) | Temperatur im Reaktor |
|---|---|
| 3 | 230°C |
| 0,75 | 230→250°C |
| 0,25 | 230→260°C |
| 0,25 | 260→270°C |
| 1 | 270°C |
| 0,5 | 270→290°C |
| 1,25 | 290°C |

Nach einer Gesamtreaktionszeit von 7 Stunden war die Umsetzung von Talg mit Ammoniak beendet. Dabei wurden gebildetes Talgfettnitril und Glycerin in zwei hintereinander geschalteten Vorlagen aufgefangen. In einer weiteren nachgeschalteten Vorlage wurde das ammoniakalische Reaktionswasser kondensiert.

Das während der Reaktion in den beiden ersten Vorlagen gesammelte Glycerin und Talgfettnitril, das in zwei Phasen anfällt, wurde getrennt und das Talgfettnitril mit Wasser nachgewaschen. So erhaltenes Talgfettnitril wurde mit 93,6% Ausbeute (413,7 g), bezogen auf Theorie, bei 1,8 Gew.-% Amidgehalt sowie 1,5 Gew.-% Talgfettsäure und Reinglycerin nach Aufarbeitung mit 80,2% (39,6 g) Ausbeute isoliert. (Hier und im folgenden sind die Ausbeutenwerte stets auf Theorie bezogen zu verstehen.)

Beispiel 2

In der in Beispiel 1 beschriebenen Apparatur wurden 495 g Rindertalg (Verseifungszahl 190, Säurezahl 1,6) zusammen mit 10 g Zink-Dodecylbenzolsulfonat vorgelet, und es wurde beginnend bei 230°C, eine Menge von 600 l $NH_3$/kg Talg · h durch das Fett geleitet. Folgender Temperaturverlauf im Reaktor wurde eingehalten:

| Reaktionszeit | Temperatur im Reaktor |
|---|---|
| 30 min | 230°C |
| 20 min | 235°C |
| 20 min | 240°C |
| 20 min | 245°C |
| 1 h 10 min | 250°C |
| 1 h 20 min | 255°C |
| 30 min | 260°C |
| 60 min | 270°C |
| 60 min | 280°C |
| 60 min | 290°C |

Die Gesamtreaktionszeit betrug 7,5 Stunden. Die in die Vorlagen ausgetragenen Reaktionsprodukte wurden mittels Wasser ausgewaschen und aufgearbeitet. Talgfettnitril wurde in einer Ausbeute von 92,3% (408 g) mit einem Amidgehalt von 0,7 Gew.-% sowie einem Fettsäuregehalt von 0,5 Gew.-% und Rohglycerin mit einer Ausbeute von 87,7% (44.7 g) erhalten. Das isolierte Rohglycerin enthielt nach der OH-Zahl von 1694 81,2% Glycerin, bezogen auf eingesetzten Talg.

Beispiel 3

In der in Beispiel 1 beschriebenen Apparatur wurden 445 g technischer Talg ("bleachable tallow") (Verseifungszahl 186, Säurezahl 12,6) und 9 g Zink-Dodecylbenzolsulfonat vorgelegt und unter den Reaktionsbedingungen von Beispiel 2 mit Ammoniak umgesetzt. Die während der Reaktion innerhalb von 7,5 Stunden ausgetragenen Reaktionsprodukte wurden vereinigt und mit Wasser Glycerin ausgewaschen. Talgfettnitril konnte nach Abtrennung der wäßrigen Phase mit 93,0% Ausbeute (370,4 g, Amidgehalt 0,5 Gew.-% und Fettsäuregehalt von 0,3 Gew.-%) isoliert werden. Aus der wäßrigen Phase wurde Rohglycerin mit 97,5% Ausbeute (38,5 g) gewonnen. Die Ausbeute an Reinglycerin betrug laut OH-Zahl von 1683 84,0%, bezogen auf eingesetzten Talg.

Beispiel 4

In die in Beispiel 1 beschriebene Apparatur, die hier mit einer 70 cm-Spiegelglaskolonne mit Raschigringen zur Fraktionierung ausgerüstet war, wurden 488 g Speisetalg (Verseifungszahl 190, Säurezahl 2,4) und 5 g Zink-Toluolsulfonat gegeben. Während des Aufheizens wurde ein schwacher Stickstoffstrom durch den Reaktor geleitet. Bei 190°C erfolgte die Umstellung auf 600 l $NH_3$/kg Fett · h. Die Umsetzung wurde 3 Stunden bei 230°C durchgeführt. Anschließend wurde innerhalb einer halben Stunde von 230°C auf 270°C hochgeheizt und für weitere 3,25 Stunden die Temperatur bei 270°C gehalten.

Nach insgesamt 6,75 Stunden waren dann alle flüchtigen Reaktionsprodukte aus dem Reaktor

über die Kolonne augetragen. Das im Vorlagensystem gesammelte Glycerin/Talgfettnitril-Gemisch wurde anschließend mit Wasser behandelt. Nach Aufarbeitung beider Phasen konnte Talgfettnitril mit einer Ausbeute von 93,2% (406 g, 1,7% Amidgehalt, 0,5 Gew.-% Fettsäuregehalt) und Reinglycerin mit einer Ausbeute von 80,1% (40,1 g) erhalten werden.

In der gleichen Apparatur und unter den gleichen Versuchsbedingungen wurde Speisetalg auch in Gegenwart weiterer Katalysatoren umgesetzt. Die Versuchsergebnisse sind nachfolgend tabellarisch angegeben:

| Katalysator | Reaktionszeit h | Nitrilausbeute % | Amidgehalt Gew.-% | Ausbeute an Reinglycerin % |
|---|---|---|---|---|
| 1 Gew.-% Zink-Perfluor-hexansulfonat | 6,25 | 93,8 | 0,9 | 78 |
| 1 Gew.-% Zink-Isopropyl-naphthalinsulfonat | 7 | 94 | 1,3 | 82 |
| 1 Gew.-% Zink-$C_{15}/C_{18}$-Alkansulfonat | 6,75 | 94 | 2 | 83 |
| 1 Gew.-% Zinksalz des Umsetzungsprodukts von Benzol, Ölsäure und $H_2SO_4$ (Alkylbenzolsulfonsäure, die in der Alkylkette eine Carboxylgruppe trägt) | 6 | 92 | 0,7 | 77 |

Beispiel 5

In der in Beispiel 4 beschriebenen Apparatur wurden 436 g Sonnenblumenöl (Verseifungszahl 189, Säurezahl 0,8) und 9 g Zink-Dodecylbenzolsulfonat als Katalysator unter den in Beispiel 4 angegebenen Bedingungen (6,75 Stunden Reaktionszeit) mit Ammoniakgas (690 l/kg Fett · h) umgesetzt. Nach Aufarbeiten wurden 94,6% Fettnitril (Amidgehalt 0,5 Gew.-%, Fettsäuregehalt 0,35 Gew.-%) und 91,2% Rohglycerin (40,9 g) bzw. laut OH-Zahl von 1717 85,6% Reinglycerin, bezogen auf eingesetztes Ol, erhalten.

Beispiel 6

Die Herstellung von Fettsäurenitril und Glycerin direkt aus Glyceriden ist auch mittels kontinuierlicher Fahrweise möglich. In einem beheizbaren, zylindrischen 750 ml Glasgefäß wurden 192 g Speisetalg zusammen mit 7,5 g Zink-Dodecylbenzolsulfonat vorgelegt. Der Reaktor war mit Rührer, Innenthermometer, beheiztem Tropftrichter und einer mit Raschigringen gefüllten 70 cm-Spiegelglaskolonne versehen. 600 l Ammoniak/kg Fett · h wurden mittels Fritte bei 230°C von unten her in den Reaktor geleitet. Unter diesen Bedingungen wurden 1009 g Speisetalg vermischt mit 1 Gew.-% Zink-Dodecylbenzolsulfonat innerhalb von 3 Stunden zudosiert (27—29g Talg/h). Die kontinuierlich über die Kolonne ausgetragenen flüchtigen Reaktionsprodukte wurden im Vorlagensystem kondensiert. Das anfallende Talgfettnitril wurde mittels Wasser von Glycerin befreit. Talgfettnitril konnte mit 96,1% Ausbeute (1033 g; 1,4 Gew.-% Amidgehalt) isoliert werden. Aus dem Wasser wurde Rohglycerin mit 95,5% Ausbeute (115,4 g) isoliert.

Beispiel 7

In der in Beispiel 4 beschriebenen Apparatur wurden 486 g Speisetalg (Verseifungszahl 190, Säurezahl 1,6) und 5 g Zink-Dodecylbenzolsulfonat vorgelegt. Bei ca. 270°C wurden konstant 500 l $NH_3$/kg Fett · h durch das Reaktionsgut geleitet. Die Reaktionszeit betrug 6,5 Stunden. Die in die Vorlagen ausgetragenen Rekationsprodukte wurden mittels Wasser gewaschen und aufgearbeitet. Talgfettnitril wurde in einer Ausbeute von 93,7% (406 g) mit einem Amidgehalt von 2,2 Gew.-% sowie einem Fettsäuregehalt von 0,4 Gew.-% und Reinglycerin mit einer Ausbeute von 73,45% (36,8 g), bezogen auf eingesetzten Talg, erhalten.

Beispiel 8

In die in Beispiel 4 beschriebene Apparatur wurden 486 g Talg (Verseifungszahl 187,5, Säurezahl 11,1) und 4,25 g Zinksalz der Talgfettsäure gegeben. Während des Aufheizens wurden 30 l $N_2$/h durch den Reaktor geleitet und bei 190°C auf 600 l $NH_3$/kg Fett · h umgestellt. Nach 13 Stunden war die Reaktion beendet. Dabei wurde die Reaktionstemperatur zunächst 3 Stunden bei 230°C und nach Aufheizen 9,5 Stunden bei 270°C gehalten. Die in den Vorlagen gesammelten, aus dem Reaktor ausgetragenen Talgfolgeprodukte wurden mit Wasser gewaschen. Talgfettnitril wurde mit 84,6%iger Ausbeute (367,8 g) und Reinglycerin mit 60,4%iger Ausbeute (28,3 g), bezogen auf eingesetzten Talg, isoliert.

Beispiel 9

In der in Beispiel 1 beschriebenen Apparatur und mit dem in Beispiel 4 angegebenen Fraktioniersystem wurden 445 g Rindertalg (Verseifungszahl 186, Säurezahl 0,8) zusammen mit 9 g Eisen-Dodecylbenzolsulfonat als Katalysator vorgelegt. Beginnend bei 230°C wurden 600 l $NH_3$/kg Fett · h durch den Talg geleitet. Die Reaktionstemperatur wurde innerhalb von 7,25 Stunden auf 290°C gesteigert. Nach Ende der Reaktion und Aufarbeitung resultierten 364,1 g Talgfettnitril (91,4% Ausbeute) mit einem Amidgehalt von 2,9 Gew.-% sowie einem Fettsäuregehalt von 0,4 Gew.-% und 30,2 reinglycerin (67,0% Ausbeute), bezogen auf eingesetzten Talg.

Beispiel 10

In die in Beispiel 1 beschriebene Apparatur wurden 445 g Rindertalg (Verseifungszahl 190, Säurezahl 0,2) und 9 g Blei-Dodecylbenzolsulfonat gegeben und dann 600 l $NH_3$/kg Fett · h, beginnend bei 230°C Reaktionstemperatur, durchgeleitet. Die Reaktionstemperatur wurde innerhalb von 7,25 Stunden kontinuierlich auf 290°C gesteigert. Nach Ende der Reaktion und Aufarbeitung resultierten 350,5 g (88,2% Ausbeute) Nitril mit einem Amidgehalt von 2,7 Gew.-% sowie einem Fettsäuregehalt von 0,35 Gew.-% und 36,5 g Reinglycerin (78,9% Ausbeute).

Beispiel 11

In der in Beispiel 1 beschriebenen Apparatur wurden 445 g Sojaöl (Verseifungszahl 203,2) zusammen mit 9 g Zink-Dodecylbenzolsulfonat vorgelegt. Beginnend bei 230°C wurden 600 l $NH_3$/kg Fett · h durch das Öl geleitet. Innerhalb von 7,25 Stunden wurde die Reaktionstemperatur auf 290°C gesteigert. Nach Ende der Reaktion und Aufarbeitung wurden 363,3 g Sojanitril (92,2% Ausbeute) mit einem Amidgehalt von 0,7 Gew.-% sowie einem Fettsäuregehalt von 0,7 Gew.-% und 36,0 g Reinglycerin (Ausbeute 72,8%, bezogen auf eingesetztes Öl) erhalten.

Beispiel 12

In der in Beispiel 1 beschriebenen Apparatur wurden 445 g Rindertalg (Verseifungszahl 192, Säurezahl 0,25) und 9 g Cadmium-Dodecylbenzolsulfonat vorgelegt. Beginnend bei 230°C wurden 600 l $NH_3$/kg Fett · h durch das Fett geleitet. Innerhalb von 6,5 Stunden wurde die Reaktionstemperatur auf 290°C gesteigert. Nach Ende der Reaktion und Aufarbeitung wurden 373,6 g Talgfettnitril (94,2% Ausbeute) mit einem Amidgehalt von 2 Gew.-% sowie einem Fettsäuregehalt von 0,7 Gew.-% und 75,2% Ausbeute an Reinglycerin (35,1 g) erhalten.

Beispiel 13

In der in Beispiel 1 beschriebenen Apparatur wurden 445 g Rindertalg (Verseifungszahl 192) und 9 g Kobalt-Dodecylbenzolsulfonat vorgelegt. Beginnend bei 230°C wurden 600 l $NH_3$/kg Fett · h durch das Fett geleitet. Innerhalb ovn 7,25 Stunden wurde die Reaktionstemperatur auf 290°C gesteigert. Nach Ende der Reaktion und Aufarbeitung resultierten 373 g (94% Ausbeute) Talgnitril mit einem Amidgehalt von 4,9 Gew.-% sowie einem Fettsäuregehalt von 0,8 Gew.-% und eine Rohglycerinausbeute von 86,4% (40,3 g).

Beispiel 14

In der in Beispiel 1 beschreibenen Apparatur, die jedoch anstelle einer Fraktioniereinheit mit einem direkten übergangsstück vom Reaktor zum Vorlagensystem ausgerüstet war, wurden 486 g Speisetalg, (Verseifungszahl 187, Säurezahl 0,8) zusammen mit 1 Gew.-% Zink-Dodecylbenzolsulfonat, bezogen auf Talg, vorgelegt. Während der Umsetzung wurden 600 l $NH_3$/kg Fett · h durch das Reaktionsgut geleitet. Es wurde dabei die Temperatur 3 Stunden bei 230°C und nach Aufheizen (0,5 Stunden) 1,25 Stunden bei 270°C gehalten Nach insgesamt 4,75 Stunden war die Reaktion beendet. Die bei 230°C und 270°C in die Vorlagen ausgetragenen Reaktionsprodukte wurden jeweils getrennt gesammelt und aufgearbeitet. Das bei 230°C isolierte Fettsäurenitril besaß einen Amidgehalt von 20 Gew.-% Bei 270°C wurde ein Amidgehalt von etwa 9 Gew.-% beobachtet. Der Amidgehalt im gesamten Fettsäurenitril war 10 Gew.-%.

Beispiel 15

In der in Beispiel 1 beschriebenen Apparatur, die jedoch anstelle einer Fraktioniereinheit mit einem direkten Übergangsstück vom Reaktor zum Vorlagensystem ausgerüstet war, wurden 500 g Talg (Verseifungszahl 189, Säurezahl 0,9) zusammen mit 2 Gew.-% Zink-Dodecylbenzolsulfonat, bezogen auf Talg, vorgelegt. Während der Umsetzung wurden 600 l $NH_3$/kg Fett · h durch das Reaktionsgut geleitet. Es wurde dabei die Temperatur 3 Stunden bei 230°C gehalten und danach um 10°C und nach je einer halben Stunde um weitere 10°C erhöht. Bei 280°C war die Reaktion beendet. Die Reaktionszeit betrug 5,25 Stunden. Das gesamte Kondensat wurde mittels Wasser bei 80°C in Fettsäurenitril und

Glycerin/Wasser aufgetrennt. Das isolierte Fettsäurenitril besaß einen Amidgehalt von 5,3 Gew.-% und einen Fettsäuregehalt von 0,5 Gew.-%. Die Nitrilausbeute betrug 95,2%. Nach Aufarbeiten konnte Reinglycerin mit einer Ausbeute von 91,4% gewonnen werden.

Beispiel 16

In einem beheizbaren Reaktor, der mit einem Gaseinleitungsrohr, einem Rührer und einem Innenthermometer ausgerüstet ist und dem ein Vorlagesystem (Kondensationssystem aus einer oder mehreren Vorlagen, Behältern) angegliedert ist, wurden 500 g technischer Talg (Verseifungszahl 191,4, Säurezahl 1,5) zusammen mit 2 Gew.-% Zink-Dodecylbenzolsulfonat, bezogen auf Talg, vorgelegt. Nun wurde auf 230°C erhitzt und 600 l Ammoniak pro kg Fett · h durch das Reaktionsgut geleitet. Die Temperatur wurde 3 Stunden bei 230°C gehalten und anschließend kontinuierlich innerhalb von 1,5 Stunden auf 260°C erhöht. Nach insgesamt 4,5 Stunden war der Austrag an Glycerin beendet und zusätzlich etwa die Hälfte an Roh-Fettsäurenitril ausgetragen. An dieser Stelle der Umsetzung wurde der Ammoniakdurchsatz auf 60 l/kg Reaktionsgut · h reduziert und die Reaktionstemperatur gleichzeitig auf 290°C gesteigert. Während des Aufheizens wurde die Wasserwäsche des glycerinhaltigen Rohnitrils durchgeführt, das dann, von Glycerin befreit, in den 290°C heißen Reaktor zurückgeführt wurde. Nach einer Reaktionszeit von insgesamt 6,5 Stunden (Nachreaktion 2 Stunden) wurde das Talgfettnitril abgelassen und durch Destillation vom Katalysator befreit. Es wurden 417 g (93,2% Ausbeute) Talgfettnitril mit einem Amidgehalt unter 0,06 Gew.-% sowie einer Säurezahl von 0,1 und (laut OH-Zahl) 42,9 g Reinglycerin (82,7% Ausbeute) erhalten.

Beispiel 17

In der in Beispiel 1 beschriebenen Apparatur wurden 500 g technischer Talg (Verseifungszahl 191,4, Säurezahl 1,5) zusammen mit 2 Gew.-% Zink-Dodecylbenzolsulfonat, bezogen auf Talg, vorbelegt. Während der Umsetzung wurden 600 l Ammoniak pro kg Fett und Stunde durch das Reaktionsgut geleitet, wobei die Temperatur 3 Stunden bei 230°C gehalten und anschließend kontinuierlich innerhalb von 2,25 Stunden bis auf 280°C erhöht wurde. Die Gesamtreaktionszeit betrug 5,25 Stunden. Während der Umsetzung wurde darauf geachtet, daß das Temperaturgefälle zwischen Sumpf und Übergang möglichst gering war. Das erhaltene Kondensat wurde in Roh-Fettsäurenitril und Glycerin mittels Wasserwäsche aufgetrennt.

Die isolierten 432,8 g Roh-Fettsäurenitril (Amidgehalt 6,1 Gew.-%) wurden in einem Reaktor, versehen mit Gaseinleitungsrohr, Rührwerk, Innenthermometer und Kondenstionssystem, vorgelegt und 2 Gew.-% Zink-Dodecylbenzolsulfonat dazugegeben. Durch das Reaktionsgemisch wurden 60 l Ammoniak pro kg Reaktionsgut · h bei 290°C durchgeleitet. Gebildetes Reaktionswasser wurde dabei ausgetragen. Die Reaktionszeit betrug 1 Stunde. Nach Destillation des Reaktorinhalts lagen 415 g Talgfettnitril (93,1% Ausbeute) mit einem Amidgehalt unter 0,05 Gew.-% und einer Säurezahl von 0,1 vor. Reinglycerin (laut OH-Zahl) wurde 47,3 g (91,2% Ausbeute) erhalten.

Vergleichsbeispiel (analog GB—PS 451,594)

Durch ein mit 800 $cm^3$ gekörntem Alumiumoxid gefülltes Glasrohr wurden von unten bei 320 bis 360°C gemeinsam 300 l $NH_3$/l Katalysator · h und 50 g Rindertalg pro Stunde durchgesetzt. Insgesamt wurden 1342 g Rindertalg zudosiert. Die flüchtigen Reaktionsprodukte, die oben aus dem Reaktor austraten, wurden in einem Vorlagensystem aufgefangen und nach ihrer Vereinigung mit Wasser gewaschen. Es wurden 1191 g rohes Talgfettnitril (Ausbeute 99%) erhalten, jedoch von schlechter Qualität (schwarz gefärbt und stechender Geruch). Glycerin konnte nicht nachgewiesen werden. Theoretisch waren 140 g Glycerin zu erwarten.

**Patentansprüche**

1. Verfahren zur Umsetzung von Mono-, Di- und Triglyceriden der allgemeinen Formel

$$\begin{array}{l} CH_2\text{—}O\text{—}(COR_1,H) \\ \mid \\ CH\text{—}O\text{—}(COR_2,H) \\ \mid \\ CH_2\text{—}O\text{—}COR_3 \end{array}$$

worin $R_1$, $R_2$ und $R_3$, die gleich oder verschieden sein können, aliphatische Kohlenwasserstoffreste mit 3 bis 23 Kohlenstoffatomen bedeuten, wobei diese Reste gegebenenfalls mit einer OH-Gruppe substituiert sein können, oder von Gemischen solcher Glyceride in flüssiger Phase mit Ammoniak unter Gewinnung von Fettsäurenitrilen der Formel $R_3$ ($R_1$, $R_2$)—CN und von Glycerin mittels Hindurchleiten von gasförmigem Ammoniak, gegebenenfalls unter Zumischung von Inertgas, bei erhöhten Temperaturen und in Gegenwart von Katalysatoren, dadurch gekennzeichnet, daß das Glycerid mit einem Ammoniakstrom von mindenstens 200 l/kg Glycerid und Stunde, bei Temperaturen von 220 bis 300°C in Gegenwart von Blei-, Zink-, Cadmium-, Zinn-, Titan-, Zirkon-, Chrom-, Antimon-, Mangan-, Eisen-, Nickel- oder

Kobaltsalzen von Carbonsäuren oder Sulfonsäuren als Katalysatoren in innigen Kontakt gebracht, das erhaltene Produktgemisch unter rascher Entfernung des gebildeten Glycerins aus der Reaktionszone ausgetragen und das ausgetragene Produktgemisch einer Phasentrennung in eine Fettsäurenitrilphase und eine Glycerin/Wasser-Phase unterworfen wird.

2. Verfahren nach Anspruch 1, dadurch gekennzeichnet, daß die Temperatur während des Reaktionsverlaufs kontinuierlich oder stufenweise über den Bereich 220 bis 300°C erhöht wird.

3. Verfahren nach Anspruch 1 und 2, dadurch gekennzeichnet, daß das Reaktionsgemisch während der Anfangsphase der Reaktion so lange bei einer Temperatur von etwa 220 bis 240°C gehalten wird, bis etwa 30 bis 70% der theoretisch zu erwartenden Glycerinmenge aus der Reaktion abgeführt worden sind, dann kontinuierlich oder stufenweise über einen Zeitraum von etwa 0,5 bis 5 Stunden auf etwa 270 bis etwa 300°C aufgeheizt und die Reaktion in diesem Temperaturbereich zu Ende geführt wird.

4. Verfahren nach Anspruch 1 bis 3, dadurch gekennzeichnet, daß die im ausgetragenen Produktgemisch enthaltenen Fettsäureamide und Fettsäuren, gegebenenfalls nach der Phasentrennung, einer Nachreaktion in Gegenwart von Ammoniak und der in Anspruch 1 definierten Katalysatoren unterworfen werden.

5. Verfahren nach Anspruch 1 bis 4, dadurch gekennzeichnet, daß das ausgetragene Produktgemisch vor der Phasentrennung einer Fraktionierung unterworfen wird, wobei im ausgetragenen Produktgemisch enthaltene Fettsäureamide und Fettsäuren in die Reaktion zurückgeführt werden.

6. Verfahren nach Anspruch 1 bis 4, dadurch gekennzeichnet, daß die Reaktion bis zur im wesentlichen vollständigen Austragung des Glycerins geführt wird, die Fettsäurenitrilphase nach Phasentrennung zu dem noch nicht ausgetragenen Fettsäurenitril zurückgeführt und zusammen mit diesem bei einer Temperatur von 200 bis 320°C in Gegenwart der in Anspruch 1 definierten Katalysatoren in einem Ammoniakstrom von 5 bis 150 l/kg Fettsäurenitril und Stunde weiterbehandelt wird.

7. Verfahren nach Anspruch 1 bis 6, dadurch gekennzeichnet, daß die Umsetzung in Gegenwart von Blei-, Cadmium-, Eisen-, kobalt- und Zinksalzen von Alkylarylsulfonsäuren mit einem oder mehreren Alkylresten mit je 1 bis 24 Kohlenstoffatomen, die mit bis zu zwei Carboxylgruppen substituiert sein können, als Katalysatoren durchgeführt wird.

## Claims

1. A process for converting mono-, di- and triglycerides of the formula

$CH_2—O—(COR_1,H)$
|
$CH—O—(COR_2,H)$
|
$CH_2—O—COR_3$

wherein $R_1$, $R_2$, and $R_3$, identical or different, are aliphatic hydrocarbon radicals or mono-hydroxy substituted hydrocarbon radicals having from 3 to 23 C atoms, or mixtures of said glycerides in the liquid phase with ammonia yielding fatty acid nitriles of the formula $R_1$ ($R_2$, $R_3$)—N and glycerol by passing gaseous ammonia optionally in admixture with an inert gas at elevated temperatures in the presence of a catalyst, which is characterised by contacting intimately the liquid glyceride with a flow of at least 200 liters ammonia per kilogram of glyceride and hour at temperatures of from 220 to 300°C in the presence of a salt of a carboxylic or sulfonic acid and a metal selected from the group of lead, zinc, cadmium, tin, titanium, zirconium, chromium, antimony, manganese, iron, nickel, and cobalt as catalysts, discharging the resulting product mixture with rapid removal of the glycerol formed from the reaction zone, and subjecting the product mixture to a phase separation into a fatty acid nitrile phase and glycerol/water phase.

2. The process according to claim 1, characterised by raising the temperature gradually or continuously from 20°C to 300°C during the course of the reaction.

3. The process according to claims 1 and 2, characterised by maintaining in the starting phase of the reaction a temperature level in the range of from about 220 to about 240°C until between 30 and 70 percent of the theoretical amount of glycerol is removed from the reaction, then raising the temperature over a period from about 0.5 to about 5 hours to a temperature level in the range of from about 270 to about 300°C and completing the reaction.

3. The process according to claims 1 to 3, characterised by subjecting fatty acids and fatty acid amides contained in said discharged product mixture to an after-treatment, optionally following the phase separation, in the presence of ammonia and of the catalysts as defined in claim 1.

5. The process according to claims 1 to 4, characterised by subjecting the discharged product mixture to fractionation prior to the phase separation whereby recycling fatty acids and fatty acid amides contained in said discharged product mixture into the reaction.

6. The process according to claims 1 to 4, characterised by continuing the reaction until substantially the entire amount of glycerol is discharged, combining the fatty acid nitrile phase

following the phase separation with the unremoved nitrile portion and treating said combined nitriles with a flow of ammonia of from 5 to 150 liters ammonia per kilogram fatty acid nitril and hour at temperatures of from 200 to 320°C in the presence of the catalysts as defined in claim 1.

7. The process according to claims 1 to 6, characterised by carrying out the reaction in the presence of lead, cadmium, iron, cobalt and zinc salts of alkylarene-sulfonic acids wherein the alkyl radicals have 1 to 24 carbon atoms, optionally substituted with one or two carboyxlic groups, as catalysts.

**Revendications**

1. Procédé de réaction de mono-, di- et tri-glycérides répondant à la formule générale:

$$CH_2—O—(COR_1,H)$$
$$|$$
$$CH—O—(COR_2,H)$$
$$|$$
$$CH_2—O—COR_3$$

dans laquelle $R_1$, $R_2$ et $R_3$, identiques ou différents, sont chacun un radical hydrocarboné aliphatique comportant de 3 à 23 atomes de carbone et éventuellement porteur d'un groupe OH, ou de mélanges de tels glycérides, en phase liquide, avec l'ammoniac de manière à obtenir des nitriles d'acides gras de formule $R_3$ ($R_1$, $R_2$)—CN et du glycérol, par passage, dans le mélange réactionnel, d'ammoniac gazeux, éventuellement mélangé à un gaz inerte, à des températures élevées et en présence de catalyseurs, procédé caractérisé en ce qu'on met le glycéride en contact intime avec un courant d'ammoniac d'au moins 200 litres par kg de glycéride et par heure, à des températures de 220 à 300°C, en présence de sels de plomb, de zinc, de cadmium, d'étain, de titane, de zirconium, de chrome, d'antimoine, de manganèse, de fer, de nickel ou de cobalt d'acides carboxyliques ou d'acides sulfoniques comme catalyseurs, on fait sortir de la zone réactionnelle le mélange produit obtenu tout en en éliminant rapidement le glycérol formé et on le soumet à une séparation de phases pour obtenir une phase de nitrile(s) d'acide(s) gras et une phase de glycérol et d'eau.

2. Procédé selon la revendication 1, caractérisé en ce qu'au cours de la réaction on élève la température, continuellement ou par étapes, entre 220 et 300°C.

3. Procédé selon l'une des revendications 1 et 2, caractérisé en ce qu'au cours de phase de début de réaction, on maintient le mélange réactionnel à une température d'environ 220 à 240°C jusqu'à avoir fait sortir du mélange réactionnel environ 30 à 70% de la quantité de glycérol a attendre en théorie, puis on chaufe continuellement ou par étapes, en l'espace d'environ 0,5 à 5 heures, à une température d'environ 270 à 300°C et on conduit dans cet intervalle de température la réaction à sa fin.

4. Procédé selon l'une quelconque des revendications 1 à 3, caractérisé en ce qu'on soumet les amides gras et les acides gras contenus dans le mélange des produits que l'on a fait sortir de la zone de réaction, et éventuellement après une séparation des phases, à une post-réaction en présence d'ammoniac et d'au moins l'un des catalyseurs définis à la revendication 1.

5. Procédé selon l'un quelconque de revendications 1 à 4, caractérisé en ce que, avant la séparation des phases, on soumet le mélange des produits que l'on a fait sortir de la zone de réaction à un fractionnement, et l'on renvoie vers la réaction les amides gras et les acides gras qui étaient contenus dans ce mélange des produits ayant quitté la zone de réaction.

6. Procédé selon l'une quelconque des revendications 1 à 4, caractérisé en ce qu'on conduit la réaction jusqu'à ce que le glycérol soit totalement éliminé, ou presque, on renvoie, après la séparation de phases, la phase des nitriles gras vers les nitriles gras n'ayant pas encore quitté la zone de réaction et l'on en continue le traitement, avec ces derniers nitriles, à une température de 200 à 320°C en présence d'au moins l'un des catalyseurs définis à la revendication 1, dans un courant d'ammoniac de 5 à 150 litres de $NH_3$ par kg de nitriles gras et par heure.

7. Procédé selon l'une quelconque des revendications 1 à 6, caractérisé en ce qu'on effectue la réaction en présence de sels de plomb, de cadmium, de fer, de cobalt, ou de zinc d'acides, alkyl-arène-sulfoniques comportant un ou plusieurs radicaux alkyles ayant chacun de 1 à 24 atomes de carbone et pouvant porter jusqu'à deux groupes carboxy comme substituants, sels qui servent de catalyseurs.